# EUROPEAN PATENT APPLICATION

(11) **EP 3 971 905 A1**
(43) Date of publication of application: **23.03.2022**
(21) Application number: 20198295.6
(22) Date of filing: 25.09.2020
(51) Int. Cl.: G16H 20/17

(54) **SYSTEM AND METHOD FOR BLOOD GLUCOSE CONTROL**

(30) Priority: 22.09.2020 KR 20200122465
(71) Applicant: Cure Stream Co., Ltd., Daegu 41585 (KR); POSTECH Research and Business Development Foundation, Pohang-si, Gyeongsangbuk-do 37673 (KR)
(72) Inventor: PARK, Sung Min, 41585 Daegu (KR); KIM, Gang Wook, 41585 Daegu (KR)
(74) Representative: Botti, Mario

(57) **Abstract**

A blood glucose control system according to an embodiment of the present disclosure includes continuous glucose monitoring (CGM) to continuously measure a patient's blood glucose level, a biosignal sensor to obtain the patient's biosignal, a lifelog data collector to collect the patient's lifelog data, an insulin infusion controller to determine an insulin dose and an infusion rate based on at least one of the blood glucose level measurement information received from the CGM and the patient's glucose metabolism characteristics information, the biosignal obtained by the biosignal sensor or the patient's stress level indexed by the biosignal, or the lifelog data collected by the lifelog data collector, and an insulin pump to deliver insulin to the patient's body according to the insulin dose and the infusion rate determined by the insulin infusion controller.

## Description

### [Technical Field]

The present disclosure relates to a system and method for blood glucose control.

### [Background Art]

Diabetes is a sort of metabolic disorder caused by insufficient or abnormal secretion of insulin, and is a disease characterized by a high concentration of glucose in blood.

Treatment methods of diabetes include nutrition therapy, exercise therapy, medication therapy, insulin injection therapy, etc., and recently methods using an artificial pancreas are being developed.

The artificial pancreas is gaining much attention from patients with diabetes who have to depend on insulin as an alternative solution capable of overcoming the limitations of the existing treatment methods.

The artificial pancreas includes Continuous Glucose Monitoring (CGM), an insulin pump patch and a control algorithm, and is configured to control the insulin pump patch using values measured from CGM by the control algorithm.

In general, the control algorithm is configured to control the insulin pump·patch to reduce a difference between a patient's current or near future blood glucose levels and a target blood glucose level based on the patient's blood glucose level information and insulin infusion history.

However, as the biological characteristics greatly differ from person to person and the characteristics of a person change over time, diabetes is a disease that is greatly affected by individuals' lifestyles such as physical activity, food intake and sleep duration, or individuals' mental health conditions (for example, stress level), and thus the existing static control algorithm not reflecting individuals' lifestyles or mental health conditions or the existing adaptive control algorithm imperfectly simulating the biological characteristics has limitations, and in particular, the control algorithm not considering individuals' lifestyles or mental health conditions has limitations in coping with changes in blood glucose level.

### [Disclosure]

### [Technical Problem]

Accordingly, there is a demand for an approach for blood glucose control with consideration for individuals' lifestyles and mental health conditions in the corresponding technical field.

### [Technical Solution]

To solve the above-described problem, an embodiment of the present disclosure provides a blood glucose control system.

The blood glucose control system includes continuous glucose monitoring (CGM) to continuously measure a patient's blood glucose level, a biosignal sensor to obtain the patient's biosignal, a lifelog data collector to collect the patient's lifelog data, an insulin infusion controller to determine an insulin dose and an infusion rate based on at least one of the blood glucose level measurement information received from the CGM and the patient's glucose metabolism characteristics information, the biosignal obtained by the biosignal sensor or the patient's stress level indexed by the biosignal, or the lifelog data collected by the lifelog data collector, and an insulin pump to deliver insulin to the patient's body according to the insulin dose and the infusion rate determined by the insulin infusion controller.

In addition, another embodiment of the present disclosure provides a blood glucose control method.

The blood glucose control method includes obtaining a patient's glucose metabolism characteristics information, obtaining the patient's blood glucose level measurement information, obtaining the patient's biosignal and lifelog data, learning the blood glucose level measurement information, the biosignal or the patient's stress level indexed based on the biosignal, the lifelog data and treatment history information based on the glucose metabolism characteristics information, and determining, by a learned model, an insulin dose and an infusion rate by an insulin pump.

Furthermore, the above-described technical solution does not enumerate all the features of the present disclosure. A variety of features of the present disclosure and their advantages and effects will be understood in further detail with reference to the following specific embodiments.

### [Advantageous Effects]

According to an embodiment of the present disclosure, it is possible to control the blood glucose level with consideration for individuals' lifestyles and mental health conditions, and through this, proactively cope with a sharp change in blood glucose level.

### [Description of Drawings]

FIG. 1 is an architecture diagram of a blood glucose control system according to an embodiment of the present disclosure.
FIG. 2 is a diagram showing a comparison of insulin activation profile for each individual.
FIG. 3 is a flowchart of a blood glucose control method according to another embodiment of the present disclosure.

### [Best Mode]

Hereinafter, the exemplary embodiments will be described in detail with reference to the accompanying drawings to allow those having ordinary skill in the technical field pertaining to the present disclosure to easily practice the present disclosure. However, in describing the exemplary embodiments of the present disclosure in detail, when it is determined that a certain detailed description of relevant known function or element may make the subject matter of the disclosed embodiments unnecessarily ambiguous, its detailed description is omitted herein. Additionally, like numerals denote like elements that perform functions and roles throughout the drawings.

In addition, throughout the specification, when an element is referred to as being 'connected to' another element, the element can be 'directly connected to' the other element, but the element may be 'indirectly connected to' the other element with intervening elements interposed between. Additionally, unless the context clearly indicates otherwise, 'comprising' specifies the presence of stated elements but does not preclude the presence or addition of one or more other elements.

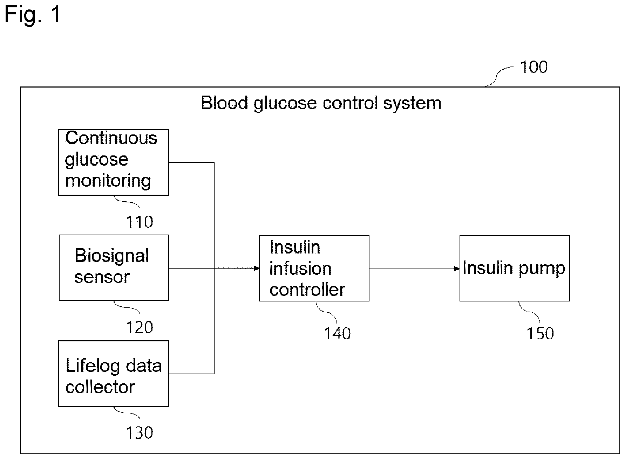

FIG. 1 is an architecture diagram of a blood glucose control system according to an embodiment of the present disclosure.

Referring to FIG. 1, the blood glucose control system 100 according to an embodiment of the present disclosure includes Continuous Glucose Monitoring (CGM) 110, a biosignal sensor 120, a lifelog data collector 130, an insulin infusion controller 140 and an insulin pump 150.

The CGM 110 may continuously measure a patient's blood glucose level, and transmit blood glucose level measurement information to the insulin infusion controller 140 via wireless communication.

According to an embodiment, the CGM 110 may include a sensor for blood glucose level measurement and a transceiver for data communication. Here, the sensor may be inserted into the patient's subcutaneous tissue to measure the glucose level in the interstitial fluid, and the transceiver may transmit the blood glucose level measurement information measured by the sensor to the insulin infusion controller 140.

The biosignal sensor 120 may obtain the patient's biosignal, and transmit the obtained biosignal to the insulin infusion controller 140 via wireless communication.

According to an embodiment, the biosignal sensor 120 may include an electrocardiogram (ECG) sensor or a heart rate sensor, and through this, may monitor the patient's cardiovascular conditions.

Additionally, the biosignal sensor 120 may include a breathing sensor, and through this, may monitor the patient's respiratory conditions.

According to another embodiment, the biosignal sensor 120 may further include a processor to analyze the obtained biosignal, and may index the patient's stress level by analysis of the biosignal obtained by the ECG sensor or the heart rate sensor and the breathing sensor through the processor. Here, a method of indexing the patient's stress level may selectively use a variety of methods known to those skilled in the art, and its detailed description is omitted herein.

The biosignal sensor 120 may be implemented in the form of a wearable sensor that can be worn on the patient, and accordingly, the biosignal sensor 120 may be worn on the patient in daily life to periodically obtain the patient's biosignal at a preset time interval and reflect it on insulin infusion control.

The lifelog data collector 130 may collect lifelog data including the patient's physical activity information, diet information and sleep information, and transmit the lifelog data to the insulin infusion controller 140 via wired or wireless communication.

According to an embodiment, the lifelog data collector 130 may be connected to an activity tracker worn on the patient to collect the patient's physical activity information (for example, physical activity volume information, etc.) measured by the activity tracker.

Additionally, the lifelog data collector 130 may be connected to a diet management application installed in, for example, the patient's mobile terminal, to collect diet information (for example, the amount of food eaten, calorie intake, etc.) inputted by the patient.

Additionally, the lifelog data collector 130 may be connected to, for example, a sleep sensor worn on the patient or placed (for example, in a bed) near the patient who is sleeping, to collect the patient's sleep information (for example, sleep pattern, sleep quality, etc.) measured and analyzed by the sleep sensor.

The insulin infusion controller 140 may determine an insulin dose and an infusion rate by the insulin pump 150 based on at least one of the blood glucose level measurement information received from the CGM 110 and the patient's glucose metabolism characteristics information, the biosignal obtained by the biosignal sensor 120 or the patient's stress level indexed by the biosignal, or the lifelog data collected by the lifelog data collector 130.

The diabetes control hormone greatly changes in working profile depending on the type and target. Accordingly, according to an embodiment of the present disclosure, it is possible to control the insulin pump 150 with consideration for glucose metabolism characteristics information that is different for each patient.

Additionally, diabetes is greatly affected by the patient's lifestyle such as physical activity, diet and sleep, or mental health conditions such as the stress level. Accordingly, according to an embodiment of the present disclosure, it is possible to determine the insulin dose more precisely by reflecting individuals' biological conditions or activities with a further consideration for at least one of lifelog data including the patient's physical activity information, diet information and sleep information, or the patient's biosignal or the patient's stress level indexed based on the biosignal to determine the insulin dose and the infusion rate in real time.

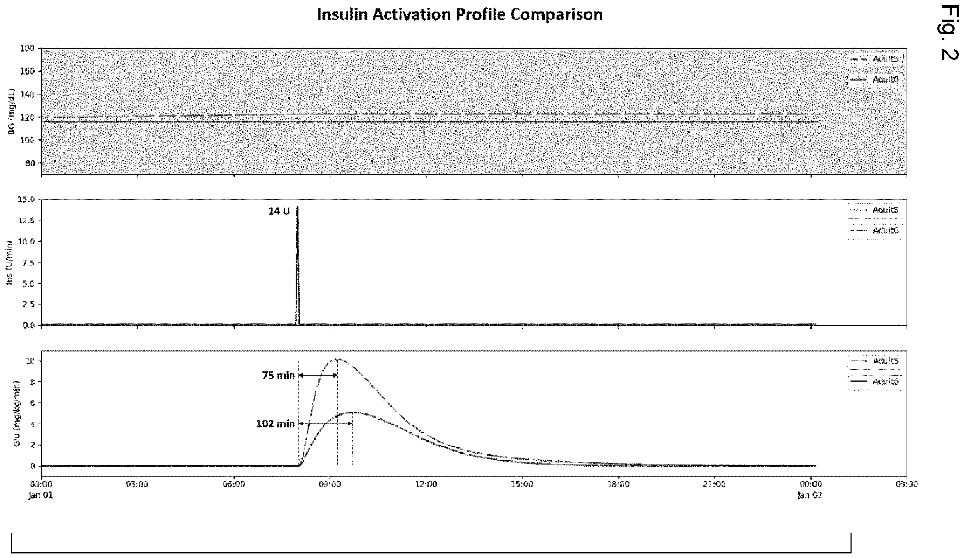

According to an example, the insulin infusion controller 140 may receive input of an insulin activation curve or insulin active peak time information obtained beforehand through testing. Here, a method of obtaining the insulin activation curve will be described with reference to FIG. 2.

For example, the insulin activation level of a patient with Type I diabetes may be measured through the following glucose clamp test.
1. Basal insulin is continuously delivered to uniformly maintain the blood glucose level of the patient on an empty stomach.
2. A preset amount of insulin (for example, 0.2 unit per unit weight) is delivered to the patient in a single dose (bolus) by subcutaneous injection.
3. When the patient's blood glucose level is lowered by the injected insulin, a glucose solution is continuously given by intravenous injection as much as a drop in blood glucose level to uniformly maintain the blood glucose level.

The infusion rate curve of the glucose solution injected into the vein in the above-described 3 becomes an Insulin Activation Curve.

FIG. 2 is a diagram showing a comparison of insulin activation profile for each individual, illustrating an Insulin Activation Curve measured through the Glucose clamp test performed on two virtual patients having similar weights.

Here, the two virtual patients Adult5 and Adult6 weigh 67.5 kg and 67.1kg respectively, and the same dose of insulin (14 Units) is delivered to them (see the second graph).

The third graph is the infusion rate curve of the glucose solution, and in other words, this is the insulin activation curve.

Referring to FIG. 2, the same dose of insulin was delivered to the two patients having similar weights, but in the case of Adult5, insulin was active at its maximum strength in 75 minutes, and in the case of Adult6, insulin was active at its maximum strength in 102 minutes, and thus it can be seen that there is a difference of about 30 minutes between the two patients in the time point at which insulin is active at its maximum strength, i.e., insulin active peak time. This is an example, and in some cases in other patients, there may be a difference of 1 hour or more in insulin active peak time.

Accordingly, according to an embodiment of the present disclosure, it is possible to optimize the blood glucose control for each individual with consideration for the insulin activation levels of individuals.

According to another embodiment, the insulin infusion controller 140 may obtain the insulin activation curve or insulin active peak time information by learning the patient's past treatment history information and past blood glucose level information. Here, the patient's treatment history information may include previous insulin infusion information (infusion time and dose).

According to still another embodiment, the insulin infusion controller 140 may obtain the insulin activation curve or insulin active peak time information by learning treatment history information and blood glucose level information of the patient who is being given insulin treatment.

Meanwhile, the insulin infusion controller 140 may continuously update the blood glucose control algorithm by repeatedly learning the blood glucose level measurement information received from the CGM 110, the biosignal received from the biosignal sensor 120 or the patient's stress level indexed based on the biosignal, the lifelog data received from the lifelog data collector 130 and the patient's past or current treatment history information, based on the patient's glucose metabolism characteristics information obtained as described above.

First, the insulin infusion controller 140 may derive additional information as described below based on the information inputted as described above, and use it to control the insulin pump 150.

In detail, when receiving the input of insulin active peak time information, the insulin infusion controller 140 may obtain the insulin activation curve from the insulin active peak time information.

Additionally, the insulin infusion controller 140 may obtain a change in blood glucose level per time from the blood glucose level measurement information received from the CGM 110, i.e., the current blood glucose level.

Additionally, the insulin infusion controller 140 may calculate the remaining insulin amount inactivated in the patient's body based on the insulin activation curve and the patient's treatment history information.

Additionally, the insulin infusion controller 140 may identify insulin dose variability from the patient's treatment history information.

Additionally, the insulin infusion controller 140 may predict an amount of glucose uptake with a further consideration for the activity volume in the lifelog data, and predict a change in blood glucose level with a further consideration for at least one of an amount of food or an amount of sleep.

Subsequently, the insulin infusion controller 140 may learn the change in blood glucose level per time, remaining insulin amount and insulin dose variability information based on the insulin activation curve, and additionally learn the amount of glucose uptake and blood glucose level change information predicted based on the lifelog data, and further, may learn the biosignal received from the biosignal sensor 120 or the patient's stress level information indexed based on the biosignal. The insulin dose and the infusion rate by the insulin pump 150 may be determined by the learned model. Here, for learning, Deep Reinforcement Learning may be applied.

As described above, according to an embodiment of the present disclosure, the amount of glucose uptake and the change in blood glucose level may be predicted based on the patient's lifelog data, and the insulin dose and the infusion rate may be determined with a further consideration for the amount of glucose uptake and the change in blood glucose level, thereby proactively coping with a sharp change in blood glucose level such as low blood glucose levels or high blood glucose levels. Additionally, the insulin dose and the infusion rate may be determined with a further consideration for the patient's mental health conditions, and thus the influence of the patient's mental health conditions on blood glucose control may be taken into consideration.

Additionally, the insulin infusion controller 140 learns as described above using the treatment history information and the blood glucose level information of the patient who is being given insulin treatment, to update the learned model to reflect the biological characteristics of a person changing over time, thereby continuously updating the blood glucose control algorithm.

In addition, the insulin infusion controller 140 may assess if insulin infusion in the past is appropriate and reflect it on learning later.

According to an embodiment, the insulin infusion controller 140 may make compensation according to the extent to which the blood glucose level is maintained, i.e., how well the blood glucose level is maintained, at a preset time interval from the insulin infusion time by the insulin pump 150 and store it for each time. In this case, the insulin infusion controller 140 may give different weights to the compensation per time according to the patient's insulin activation curve. For example, immediately after insulin infusion, the weight may be set to 0, and a greatest weight may be given to the insulin active peak time in the insulin activation curve. Subsequently, the insulin infusion controller 140 may assess if insulin infusion is appropriate by summing up the weighted compensation (i.e., compensation * weight) per time for a preset period of time (for example, 8 hours) from the insulin infusion time.

The insulin infusion controller 140 may assess if insulin infusion is appropriate for each insulin infusion by the insulin pump 150, and reflect it on the learning algorithm.

The insulin pump 150 may deliver insulin to the patient's body according to the insulin dose and the infusion rate determined by the insulin infusion controller 140.

According to an embodiment of the present disclosure as described above, for insulin infusion control, it is possible to control the insulin infusion more precisely and accurately with a comprehensive consideration for data reflecting the patient's past conditions including the patient's insulin activation level or the patient's treatment history information and blood glucose level information, as well as the patient's current conditions including the patient's biosignal or lifelog data.

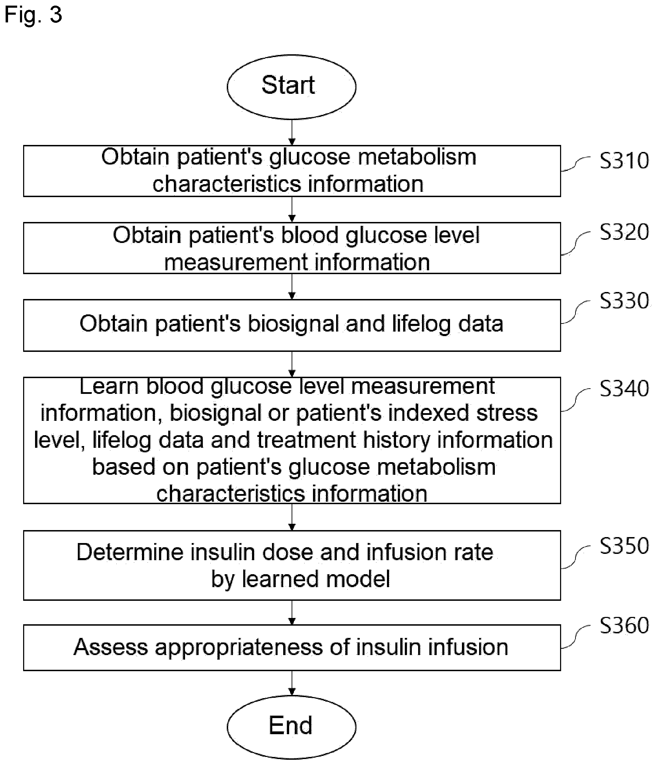

FIG. 3 is a flowchart of a blood glucose control method according to another embodiment of the present disclosure.

Referring to FIG. 3, first, the patient's glucose metabolism characteristics information may be obtained (S310). Here, the glucose metabolism characteristics information may include an insulin activation curve or insulin active peak time information.

Subsequently, the patient's blood glucose level measurement information may be obtained (S320). Here, the patient's blood glucose level measurement information may be continuously measured by the CGM.

Subsequently, the patient's biosignal and lifelog data may be obtained (S330). Here, the patient's biosignal may be a signal obtained by the ECG sensor or the heart rate sensor, and/or the breathing sensor, and the patient's lifelog data may include the patient's physical activity information, diet information and sleep information.

Subsequently, a learning process is performed to learn the blood glucose level measurement information, the biosignal or the patient's indexed stress level, lifelog data and treatment history information based on the patient's glucose metabolism characteristics information (S340), and the insulin dose and the infusion rate by the insulin pump may be determined by the learned model (S350).

Subsequently, appropriateness of insulin infusion by the insulin pump may be assessed (S360).

The blood glucose control method shown in FIG. 3 may be performed by a controller having a learning engine.

Additionally, a detailed description of each step described above is the same as those described with reference to FIG. 1, and redundant descriptions are omitted herein.

The present disclosure is not limited to the above-described embodiments and the accompanying drawings. It will be obvious to those having ordinary skill in the technical field pertaining to the present disclosure that substitutions, modifications and changes may be made to the elements of the present disclosure without departing from the technical spirit of the present disclosure.

### [Detailed Description of Main Elements]

100: Blood glucose control system
110: Continuous glucose monitoring
120: Biosignal sensor
130: Lifelog data collector
140: Insulin infusion controller
150: Insulin pump

## Claims

1. A blood glucose control system, comprising:
continuous glucose monitoring (CGM) to continuously measure a patient's blood glucose level;
a biosignal sensor to obtain the patient's biosignal;
a lifelog data collector to collect the patient's lifelog data;
an insulin infusion controller to determine an insulin dose and an infusion rate based on at least one of the blood glucose level measurement information received from the CGM and the patient's glucose metabolism characteristics information, the biosignal obtained by the biosignal sensor or the patient's stress level indexed by the biosignal, or the lifelog data collected by the lifelog data collector; and
an insulin pump to deliver insulin to the patient's body according to the insulin dose and the infusion rate determined by the insulin infusion controller.

2. The blood glucose control system according to claim 1, wherein the biosignal sensor includes an electrocardiogram (ECG) sensor or a heart rate sensor, and monitors the patient's cardiovascular conditions.

3. The blood glucose control system according to claim 2, wherein the biosignal sensor includes a breathing sensor, and monitors the patient's respiratory conditions.

4. The blood glucose control system according to claim 3, wherein the biosignal sensor includes a processor, and indexes the patient's stress level by analysis of the patient's biosignal through the processor.

5. The blood glucose control system according to claim 1, wherein the lifelog data collector is connected to an activity tracker worn on the patient to collect the patient's physical activity information measured by the activity tracker.

6. The blood glucose control system according to claim 1, wherein the lifelog data collector is connected to a diet management application installed in the patient's mobile terminal to collect diet information inputted by the patient.

7. The blood glucose control system according to claim 1, wherein the lifelog data collector is connected to a sleep sensor worn on the patient or placed near the patient who is sleeping to collect the patient's sleep information measured and analyzed by the sleep sensor.

8. The blood glucose control system according to claim 1, wherein the patient's glucose metabolism characteristics information includes an insulin activation curve or insulin active peak time information.

9. A blood glucose control method, comprising:
obtaining a patient's glucose metabolism characteristics information;
obtaining the patient's blood glucose level measurement information;
obtaining the patient's biosignal and lifelog data;
learning the blood glucose level measurement information, the biosignal or the patient's stress level indexed based on the biosignal, the lifelog data and treatment history information based on the glucose metabolism characteristics information; and
determining, by a learned model, an insulin dose and an infusion rate by an insulin pump.

10. The blood glucose control method according to claim 9, further comprising: assessing appropriateness of the insulin infusion by the insulin pump.
